# EUROPEAN PATENT APPLICATION

(11) **EP 2 952 228 A1**
(43) Date of publication of application: **09.12.2015**
(21) Application number: 14745358.3
(22) Date of filing: 27.01.2014
(51) Int. Cl.: A61N 7/02

(54) **ULTRASONIC TREATMENT HEAD AND ULTRASONIC TREATMENT DEVICE**

(30) Priority: 29.01.2013 CN 201310034077
(71) Applicant: Chongqing Haifu Medical Technology Co., Ltd., Renhe Town Yubei District Chongqing 401121 (CN)
(72) Inventor: HU, Diyuan, Chongqing 401121 (CN); ZENG, Tao, Chongqing 401121 (CN); HUANG, Guanghua, Chongqing 401121 (CN)
(74) Representative: Cohausz & Florack
(86) International application number: PCT/CN2014/071570
(87) International publication number: WO 2014/117714

(57) **Abstract**

The present invention provides an ultrasonic treatment head. The ultrasonic treatment head comprises a casing and an acoustically transparent window. An ultrasonic transmitting unit is provided in the casing; an opening, which is closed by the acoustically transparent window, is formed at one end of the casing; and the ultrasonic transmitting unit transmits ultrasonic waves to the outside through the acoustically transparent window, wherein the acoustically transparent window is made of acoustically transparent soft material. Correspondingly, the present invention further provides an ultrasonic treatment device comprising the ultrasonic treatment head. When a patient is treated by using the ultrasonic treatment head and the ultrasonic treatment equipment of the present invention, comfortable sense of the patient can be remarkably improved.

## Description

### Technical field of the Invention

The present invention belongs to the technical field of ultrasonic treatment, and relates to an ultrasonic treatment head and ultrasonic treatment equipment comprising the ultrasonic treatment head.

### Background of the Invention

At present, treatments to soft-tissue injury pain in the head and neck, the lower back, and the limbs mainly include acupuncture treatment, drug treatment and ultrasonic treatment. With regard to the ultrasonic treatment, ultrasonic equipment simulating functions of acupuncture may be employed at present, for example, ultrasonic acupuncture equipment. The ultrasonic acupuncture equipment can simulate acupuncture manipulation in traditional acupuncture, and the working principle thereof is as follows: allowing ultrasonic waves to pass through skin and converging under the skin, and simulating the acupuncture manipulation by using sound energy generated by the convergence to stimulate human body acupoints, in order to achieve the effect of traditional acupuncture. In the existing ultrasonic acupuncture equipment, a part for contacting with the human body is usually made of a solid substance such as glass, copper, aluminum or the like, consequently resulting in the defects such as unstable energy output, too fast surface temperature rise, which causes such safety hazards that patients' skin is easy to be scalded and the like, and hard texture, which makes patients feel cold and uncomfortable when initially used in cold weather and thus results in low comfort level.

### Summary of the Invention

The technical problem to be solved by the present invention is to provide an ultrasonic treatment head and ultrasonic treatment equipment comprising the ultrasonic treatment head, in view of the above deficiencies in the prior art. By using the ultrasonic treatment head provided in the present invention, the comfortable sense of patients can be improved remarkably during the treatment.

A technical solution employed to solve the technical problem of the present invention is an ultrasonic treatment head, which includes a casing and an acoustically transparent window, an ultrasonic transmitting unit being provided in the casing, an opening closed by the acoustically transparent window being formed at one end of the casing, and the ultrasonic transmitting unit transmitting ultrasonic waves to the outside through the acoustically transparent window, wherein the acoustically transparent window is made of acoustically transparent soft material.

Preferably, the acoustically transparent window is convex without any dead angle for cleaning, and thus being convenient for cleaning and disinfecting.

Preferably, an ultrasonic conducting medium is further included in the casing, the ultrasonic conducting medium being arranged between the ultrasonic transmitting unit and the acoustically transparent window and being a soft substance.

Preferably, the ultrasonic conducting medium is gelatinous or liquid.

Preferably, a sealed chamber is formed among the acoustically transparent window, the ultrasonic transmitting unit and the casing, and the ultrasonic conducting medium is arranged in the sealed chamber.

Further preferably, a bore, through which the ultrasonic conducting medium can be filled into the sealed chamber, is formed in the middle of the ultrasonic transmitting unit.

More preferably, a sealing unit configured to seal the ultrasonic transmitting unit against the casing is further included in the casing.

Preferably, the sealing unit includes a sealing ring provided between the casing and the ultrasonic transmitting unit, and a pressing mechanism, which causes deformation of the sealing ring and thus to seal the ultrasonic transmitting unit against the casing; or
the sealing unit includes a sealant provided between the casing and the ultrasonic transmitting unit, the sealant being a silicone rubber or silicone sealant or AB glue; and/or, the sealing unit includes a sealing plug for sealing the bore in the middle of the ultrasonic transmitting unit.

A heat radiating unit configured to cool the ultrasonic transmitting unit is further included in the casing.

Preferably, the heat radiating unit includes an air inlet and an air outlet both formed on the casing, and a fan provided in the casing, wherein the air inlet and the air outlet are formed on different sides of the casing, respectively; cold air outside comes into the casing from the air inlet, then passes through the fan and is exhausted from the air outlet to take away heat generated by the ultrasonic transmitting unit during working.

Further preferably, the heat radiating unit further includes a heat conducting layer applied to the back of the ultrasonic transmitting unit.

Preferably, the heat conducting layer is hygroscopic heat conducting material, which is uniformly applied to the back of the ultrasonic transmitting unit, and a metal backing is provided on the back of the ultrasonic transmitting unit.

Preferably, the air outlet is arranged nearby the acoustically transparent window, thereby avoiding the influence of exhausted hot gas on operators during treatment. Meanwhile, warm air spread around the treatment area of a patient improves the comfortable sense of the patient, promotes the blood circulation, and consequently plays a role of auxiliary therapy to some extent.

Alternatively, preferably, the ultrasonic conducting medium is liquid; the heat radiating unit includes an ultrasonic conducting medium supply mechanism, and a liquid inlet tube and a liquid return tube both connected between the ultrasonic conducting medium supply mechanism and the sealed chamber, and the ultrasonic conducting medium circulates, through the liquid inlet tube and the liquid return tube, between the ultrasonic conducting medium supply mechanism and the sealed chamber, so as to take away heat generated by the ultrasonic transmitting unit during working.

Preferably, the acoustically transparent window is made of polyurethane elastomer or polytetrafluoroethylene, and thickness of the acoustically transparent window is less than 2 mm.

Preferably, frequency of ultrasonic waves transmitted by the ultrasonic transmitting unit ranges from 0.3 MHz to 3 MHz, power thereof ranges from 0.1 W to 10 W, and average sound intensity thereof in spatial peak time ranges from 0.1 w/cm² to 40 w/cm²; width of a focal region formed by the ultrasonic waves ranges from 2 mm to 5 mm, length of the focal region is 3 to 12 times the width thereof, and focal plane distance ranges from 3 mm to 80 mm; height of the ultrasonic transmitting unit is in the range of 3 mm to 20 mm, thickness thereof is in the range of 0.5 mm to 10 mm, outer diameter thereof is in the range of 10 mm to 100 mm, and radius of an inner sphere thereof is in the range of 10 mm to 100 mm.

The present invention further provides a ultrasonic treatment equipment including an ultrasonic treatment head, which is the ultrasonic treatment head described above.

As the acoustically transparent window in the ultrasonic treatment head of the present invention is made of acoustically transparent soft material and the ultrasonic conducting medium is also a soft substance, the part of the ultrasonic treatment head for contacting with the human body is soft in texture and comfortable in touching; moreover, the surface temperature of the treatment head is not too low, resulting in a warm feeling during the treatment, thus the ultrasonic treatment head is high seasonal adaptable and applicable to both summer and winter, can effectively improve the comfortable sense of patients, further ease patients' pain during the treatment. Meanwhile, the safety of treatment is improved.

The ultrasonic treatment head of the present invention is particularly suitable for treating soft-tissue injury pain in the head and neck, the lower back, the limbs and other parts.

### Brief Description of the Drawings

Fig. 1 is a structural principle diagram of an ultrasonic treatment head according to a first embodiment of the present invention;
Fig. 2 is a structural schematic diagram of an ultrasonic treatment head according to a second embodiment of the present invention;
Fig. 3 is a structural schematic diagram of an ultrasonic treatment head according to a third embodiment of the present invention; and
Fig. 4 is a structural schematic diagram of a pressing mechanism according to the second Embodiment of the present invention.

In the drawings:
10: acoustically transparent window;
20: ultrasonic conducting medium;
30: ultrasonic transmitting unit;
31: bore;
40: casing;
41: air outlet;
42: air inlet;
50: cable;
51: liquid inlet tube;
52: liquid return tube;
60: impedance matching unit;
70: treatment head control unit;
80: fan;
90: sealing unit;
91: sealing plug;
92: sealing ring;
93: pressing mechanism;
94: spinning ring;
95: pressing sleeve.

### Detailed Description of the Embodiments

To make those skilled in the art understand the technical solutions of the present invention better, the present invention will be further described in detail below with reference to the accompanying drawings and specific embodiments.

### First Embodiment

This embodiment provides ultrasonic treatment equipment, which includes an ultrasonic treatment head.

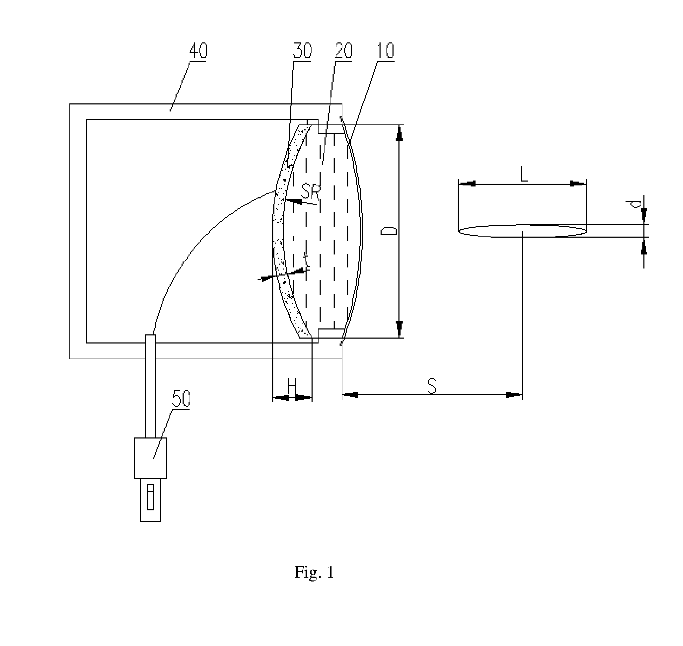

As shown in Fig. 1, the ultrasonic treatment head in this embodiment includes a casing 40 and an acoustically transparent window 10, an ultrasonic transmitting unit 30 is provided in the casing 40, an opening, which is closed by the acoustically transparent window 10, is formed at one end of the casing 40, the ultrasonic transmitting unit 30 transmits ultrasonic waves to the outside through the acoustically transparent window 10, and the acoustically transparent window 10 is made of acoustically transparent soft material.

### Second Embodiment

This embodiment provides ultrasonic treatment equipment, which includes a main machine and an ultrasonic treatment head connected with the main machine through a cable 50. The cable 50 is configured to transmit ultrasonic wave driving signals and control signals both transmitted by the main machine to the ultrasonic treatment head.

In this embodiment, the ultrasonic treatment equipment is specifically ultrasonic acupuncture equipment mainly configured to treat soft tissue injuries and muscle pains.

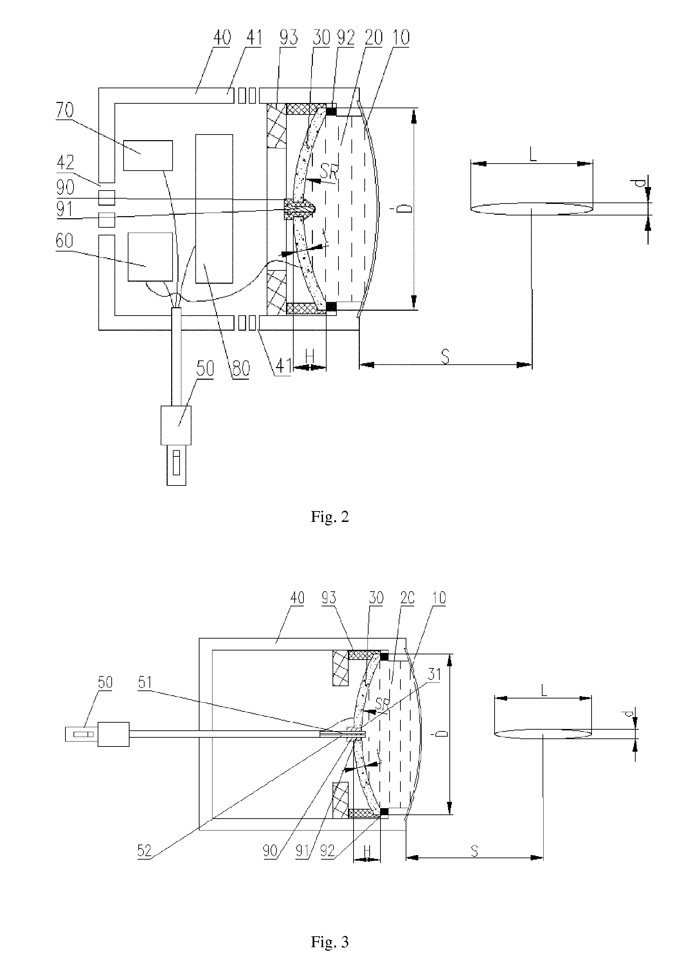

As shown in Fig. 2, the ultrasonic treatment head in this embodiment includes a casing 40 and an acoustically transparent window 10. An opening closed by the acoustically transparent window 10 is formed at one end of the casing 40, and an ultrasonic transmitting unit 30, a treatment head control unit 70, a sealing unit 90 and an ultrasonic conducting medium 20 (i.e., a coupling medium) are provided in the casing 40. The ultrasonic transmitting unit 30 is configured to transmit ultrasonic waves; the treatment head control unit 70 is configured to control output and output stopping of the ultrasonic waves. The ultrasonic transmitting unit 30 is arranged at an end in the casing close to the acoustically transparent window 10, the ultrasonic conducting medium 20 is between the ultrasonic transmitting unit 30 and the acoustically transparent window 10, and the ultrasonic transmitting unit 30 is sealed against the casing 40 through the sealing unit 90. As the open end of the casing is sealed by the acoustically transparent window 10, a sealed chamber is formed among the acoustically transparent window 10, the ultrasonic transmitting unit 30 and the casing 40, and the ultrasonic conducting medium 20 is in the sealed chamber. The acoustically transparent window 10 comes into contact with the skin of a patient, and the ultrasonic transmitting unit 30 transmits ultrasonic waves to the skin of the patient through the ultrasonic conducting medium 20 and the acoustically transparent window 10. In addition, a bore 31 may also be formed in the middle of the ultrasonic transmitting unit 30, and the ultrasonic conducting medium 20 may be filled into the sealed chamber through the bore 31. Of course, the bore 31 may be formed in other positions on the ultrasonic transmitting unit 30, which is not limited.

The acoustically transparent window 10 is made of acoustically transparent soft material. In this embodiment, the acoustically transparent window 10 is made of polyurethane elastomer (TPU) or polytetrafluoroethylene, both of which are excellent acoustically transparent material. Furthermore, the thickness of the acoustically transparent window 10 is set to be less than 2 mm.

Preferably, the ultrasonic conducting medium 20 is a soft substance. Specifically, in this embodiment, the ultrasonic conducting medium 20 is made of gelatinous acoustically transparent material, and the gelatinous ultrasonic transparent material has properties of good ultrasound penetrability and small energy loss. The ultrasonic conducting medium 20 is within the sealed chamber, which can prevent outflow of the gelatinous acoustically transparent material, to avoid influencing ultrasonic transmission. As the acoustically transparent window is made of acoustically transparent soft material and the thickness of the acoustically transparent window 10 is less than 2 mm, and meanwhile, the ultrasonic conducting medium 20 is made of gelatinous acoustically transparent material, the part of the ultrasonic treatment head for contacting with the human body is soft in texture, thereby solving the problem of insufficient comfort level during the treatment; furthermore, the surface temperature of the acoustically transparent window 10 is not too low, resulting in a warm feeling during the treatment, thus the ultrasonic treatment head is high seasonal adaptable and applicable to both summer and winter, and can effectively improve the comfortable sense of patients during the treatment.

Preferably, the sealing unit includes a sealing ring 92 arranged between the casing 40 and the ultrasonic transmitting unit 30, and a pressing mechanism 93 which can cause deformation of the sealing ring 92 and thus to seal the ultrasonic transmitting unit 30 against the casing 40.

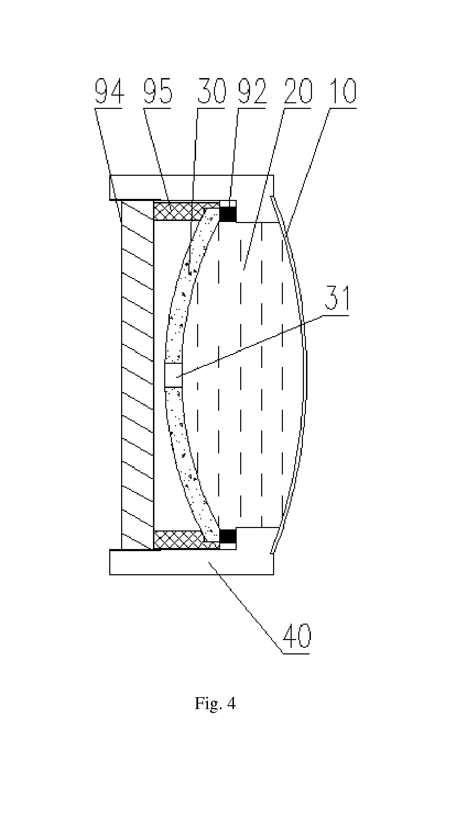

Specifically, in this embodiment, as shown in Fig. 4, the pressing mechanism 93 includes a spinning ring 94 and a pressing sleeve 95. The spinning ring 94 is arranged in the casing 40, and the pressing sleeve 95 is connected with the spinning ring 94 and comes into contact with the back of the ultrasonic transmitting unit 30. The pressing sleeve 95 may be driven to move toward the acoustically transparent window 10 by rotating the spinning ring 94, so as to compress the sealing ring 92, so that the sealing ring 92 is deformed and thus seal the ultrasonic transmitting unit 30 against the casing 40.

Preferably, the sealing unit further includes a sealing plug for sealing the bore 31 in the middle of the ultrasonic transmitting unit, and the sealing plug may be a silicone rubber or silicone sealant or AB glue.

Alternatively, the sealing unit may include a sealant provided between the casing 40 and the ultrasonic transmitting unit 30, and a gap between the both is filled with the sealant, so as to form a seal. The sealant may be a silicone rubber or silicone sealant or AB glue.

Preferably, an impedance matching unit 60 is further included in the casing 40. A cable 50 is sequentially connected to the impedance matching unit 60 and the ultrasonic transmitting unit 30. The matching problem between the individual impedance difference of the ultrasonic transmitting unit 30 and a drive signal source can be solved through impedance matching of the impedance matching unit 60.

Preferably, a heat radiating unit configured to cool the ultrasonic transmitting unit 30 is further included in the casing.

In this embodiment, the heat radiating unit includes an air inlet 42 and an air outlet 41 both provided on the casing 40, and a fan 80 provided in the casing 40. The fan 80 is arranged in a position rightly opposite to the back of a transmitting surface of the ultrasonic transmitting unit. Cold air outside comes into the casing 40 from the air inlet 42, then passes through the fan 80 and is exhausted from the air outlet 41, so as to take away heat generated by the ultrasonic transmitting unit 30 during working.

In this embodiment, the air outlet 41 is arranged close to the acoustically transparent window 10, thereby avoiding the influence of exhausted hot gas on operators during the treatment. Meanwhile, warm air spread around the treatment area of a patient improves the comfortable sense of the patient, promotes the blood circulation, and consequently plays a role of auxiliary therapy to some extent.

Preferably, the heat radiating unit further includes a heat conducting layer applied to the back of the ultrasonic transmitting unit. Specifically, the heat conducting layer may be hygroscopic heat conducting material, which is uniformly applied to the back of the ultrasonic transmitting unit.

Preferably, a metal backing may be further provided on the back of the ultrasonic transmitting unit, in order to further improve the heat radiating effect and ensure the stable output of the ultrasonic transmitting unit 30, thus improving the safety of treatment.

In this embodiment, the height H of the used ultrasonic transmitting unit 30 is in the range of 3 mm to 20 mm, the thickness t thereof is in the range of 0.5 mm to 10 mm, the outer diameter D thereof is in the range of 10 mm to 100 mm, and the radius SR of an inner sphere thereof is in the range of 10 mm to 100 mm.

The frequency of ultrasonic waves transmitted by the ultrasonic transmitting unit 30 ranges from 0.3 MHz to 3 MHz, the power thereof ranges from 0.1 W to 10 W, and the average sound intensity thereof in spatial peak time ranges from 0.1 w/cm² to 40 w/cm²; the width d of a focal region formed by the ultrasonic waves ranges from 2 mm to 5 mm, the length L of the focal region ranges from 3d to 12d, and the focal plane distance S ranges from 3 mm to 80 mm.

### Third Embodiment

As shown in Fig. 3, the difference between the ultrasonic treatment head in this embodiment and that in the second embodiment lies in that, the ultrasonic conducting medium 20 is liquid, and the structure of the heat radiating unit is also different.

In this embodiment, the ultrasonic conducting medium 20 is acoustically transparent liquid material, for example, may be water, refrigerating fluid or oil.

In this embodiment, the heat radiating unit includes an ultrasonic conducting medium supply mechanism (not shown in Fig. 3), and a liquid inlet tube 51 and a liquid return tube 52 both connected between the ultrasonic conducting medium supply mechanism and the sealed chamber. A bore 31 is formed in the middle of the ultrasonic transmitting unit 30, but the bore 31 is plugged by a sealing plug 91, in which a bore path is provided. Specifically, the liquid inlet tube 51 and the liquid return tube 52 pass through the bore path in the sealing plug 91 and the bore 31 in the ultrasonic transmitting unit 30 to connect the ultrasonic conducting medium supply mechanism with the sealed chamber, so that the liquid inlet tube 51 and the liquid return tube 52 allow circulation between the ultrasonic conducting medium supply mechanism and the sealed chamber, so as to take away heat generated by the ultrasonic transmitting unit 30 during working.

Other structures of the ultrasonic treatment head in this embodiment are the same as those in the second embodiment, and will not be repeated here.

It may be understood that, the above embodiments are exemplary embodiments merely intended to illustrate the principle of the present invention, but the present invention is not limited thereto. For a person having ordinary skill in the art, various variations and improvements may be made without departing from the spirit and essence of the present invention, and those variations and improvements should also be regarded as being within the protection scope of the present invention.

## Claims

1. An ultrasonic treatment head, comprising a casing and an acoustically transparent window, wherein an ultrasonic transmitting unit is provided in the casing, an opening, which is closed by the acoustically transparent window, is formed at one end of the casing, and the ultrasonic transmitting unit transmits ultrasonic waves to outside through the acoustically transparent window, **characterized in that** the acoustically transparent window is made of acoustically transparent soft material.

2. The ultrasonic treatment head according to claim 1, **characterized in that** an ultrasonic conducting medium is further comprised in the casing, wherein the ultrasonic conducting medium is arranged between the ultrasonic transmitting unit and the acoustically transparent window and is a soft substance.

3. The ultrasonic treatment head according to claim 2, **characterized in that** the ultrasonic conducting medium is gelatinous or liquid.

4. The ultrasonic treatment head according to claim 2, **characterized in that** a sealed chamber is formed among the acoustically transparent window, the ultrasonic transmitting unit and the casing, and the ultrasonic conducting medium is arranged in the sealed chamber.

5. The ultrasonic treatment head according to claim 4, **characterized in that** a bore, through which the ultrasonic conducting medium can be filled into the sealed chamber, is formed in the middle of the ultrasonic transmitting unit.

6. The ultrasonic treatment head according to claim 5, **characterized in that** a sealing unit configured to seal the ultrasonic transmitting unit against the casing is further comprised in the casing.

7. The ultrasonic treatment head according to claim 6, **characterized in that** the sealing unit comprises a sealing ring provided between the casing and the ultrasonic transmitting unit, and a pressing mechanism, which causes deformation of the sealing ring and thus to seal the ultrasonic transmitting unit against the casing;
or
the sealing unit comprises a sealant provided between the casing and the ultrasonic transmitting unit, the sealant being a silicone rubber or silicone sealant or AB glue; and/or, the sealing unit comprises a sealing plug for sealing the bore in the middle of the ultrasonic transmitting unit.

8. The ultrasonic treatment head according to claim 6, **characterized in that** a heat radiating unit configured to cool the ultrasonic transmitting unit is further comprised in the casing.

9. The ultrasonic treatment head according to claim 8, **characterized in that** the heat radiating unit comprises an air inlet and an air outlet both formed on the casing, and a fan provided in the casing, wherein the air inlet and the air outlet are formed on different sides of the casing, respectively, and cold air outside comes into the casing from the air inlet, then passes through the fan and is exhausted from the air outlet to take away heat generated by the ultrasonic transmitting unit during working.

10. The ultrasonic treatment head according to claim 9, **characterized in that** the heat radiating unit further comprises a heat conducting layer applied to back of the ultrasonic transmitting unit.

11. The ultrasonic treatment head according to claim 10, **characterized in that** the heat conducting layer is hygroscopic heat conducting material, which is uniformly applied to the back of the ultrasonic transmitting unit, and a metal backing is provided on the back of the ultrasonic transmitting unit.

12. The ultrasonic treatment head according to claim 8, **characterized in that** the ultrasonic conducting medium is liquid; the heat radiating unit comprises an ultrasonic conducting medium supply mechanism, and a liquid inlet tube and a liquid return tube both connected between the ultrasonic conducting medium supply mechanism and the sealed chamber, and the ultrasonic conducting medium circulates, through the liquid inlet tube and the liquid return tube, between the ultrasonic conducting medium supply mechanism and the sealed chamber, so as to take away heat generated by the ultrasonic transmitting unit during working.

13. The ultrasonic treatment head according to any one of claims 1-12, **characterized in that** the acoustically transparent window is made of polyurethane elastomer or polytetrafluoroethylene, and thickness of the acoustically transparent window is less than 2 mm.

14. The ultrasonic treatment head according to any one of claims 1-12, **characterized in that** frequency of ultrasonic waves transmitted by the ultrasonic transmitting unit ranges from 0.3 MHz to 3 MHz, power thereof ranges from 0.1 W to 10 W, and average sound intensity thereof in spatial peak time ranges from 0.1 w/cm² to 40 w/cm²; width of a focal region formed by the ultrasonic waves ranges from 2 mm to 5 mm, length of the focal region is 3 to 12 times the width thereof, and focal plane distance thereof ranges from 3 mm to 80 mm; height of the ultrasonic transmitting unit is in the range of 3 mm to 20 mm, thickness thereof is in the range of 0.5 mm to 10 mm, outer diameter thereof is in the range of 10 mm to 100 mm, and radius of an inner sphere thereof is in the range of 10 mm to 100 mm.

15. An ultrasonic treatment equipment, comprising an ultrasonic treatment head, **characterized in that** the ultrasonic treatment head is the ultrasonic treatment head according to any one of claims 1-14.
